# EUROPÄISCHE PATENTSCHRIFT

(11) **EP 1 454 584 B1**
(45) Veröffentlichungstag und Bekanntmachung des Hinweises auf die Patenterteilung: **23.05.2007**
(21) Anmeldenummer: 04003930.7
(22) Anmeldetag: 20.02.2004
(51) Int. Cl.: A61B 5/103

(54) **Messvorrichtung mit einer auf Messzellen gelagerten Tragplatte für eine Person**
Measuring apparatus with a support plate mounted upon measuring cells for a person
Dispositif de mesure équipé d'une plaque de soutient montée sur des cellules de mesure pour une personne

(30) Priorität: 04.03.2003 DE 10309567
(43) Veröffentlichungstag der Anmeldung: 08.09.2004
(73) Patentinhaber: Otto Bock HealthCare GmbH, 37115 Duderstadt (DE)
(72) Erfinder: Blumentritt, Siegmar Dr., 37136 Bösinghausen (DE); Hollaschke, Walter, 37115 Duderstadt (DE); Klingebiel, Karl-Heinz, 37115 Duderstadt (DE)
(74) Vertreter: Lins, Edgar

(56) Entgegenhaltungen:
- FR-A- 2 802 795
- US-A- 5 609 162

## Beschreibung

Die Erfindung betrifft eine Messvorrichtung mit einer auf Messzellen gelagerten Tragplatte für eine Person und einer Anzeigeeinrichtung zur Anzeige einer Kraftwirkungslinie für eine auf Auflageflächen oberhalb der Tragplatte stehenden Person.

Die Korrektur orthopädischer Fehlhaltungen, die durch die unteren Extremitäten bedingt sind, erfolgt herkömmlich durch eine Beobachtung der Körpergeometrie. So wird beispielsweise eine Beinlängendifferenz aufgrund der Stellung des Bekkens und des Verlaufs der Lendenwirbelsäule beurteilt. Diese Beurteilung ist nur grob qualitativ und auch nicht fehlerfrei möglich. Etwaige Korrekturmaßnahmen werden dadurch bestimmt, dass beispielsweise für einen Beinlängenausgleich Höhenausgleichsbrettchen verwendet werden, mit denen die Wirkung etwaiger Korrekturmaßnahmen durch Überprüfung der Beckenposition und/oder des Wirbelsäulenverlaufs bestimmt wird.

Eine quantitative Messungen erlaubende Messvorrichtung der eingangs erwähnten Art ist durch EP 0 663 181 A1 bekannt geworden. Durch unterhalb beispielsweise der Ecken einer rechteckigen Tragplatte angeordnete Druck- oder Kraftsensoren wird bei der bekannten Messvorrichtung eine Kraftwirkungslinie ermittelt und angezeigt, beispielsweise dadurch, dass der Verlauf einer Schwerpunktsebene auf den Körper der auf der Tragplatte stehenden Person oder der Verlauf einer Kraftwirkungslinie an einem mit einer Prothese versehenen Bein mittels eines vertikal oszillierenden Laserstrahls projiziert wird. Eine derartige Messvorrichtung eignet sich für die Anzeige des Verlaufs der Schwerpunktsebene sowohl bezüglich der Frontalebene als auch der Sagittalebene der auf der Tragplatte stehenden Person. Die bekannte Messvorrichtung dient zur Anpassung von Prothesen oder Orthesen der unteren Extremitäten.

Die bekannte Messvorrichtung erlaubt eine objektivierte Beurteilung des Kräfteverlaufs des Stützapparats eines menschlichen Körpers und insbesondere eine praxisgerechte Anpassung von Prothesen und Orthesen. Die Wirkung von Korrektur- bzw. Optimierungsmaßnahmen wird dadurch überprüft, dass nach der durchgeführten Korrektur- oder Optimierungsmaßnahme eine erneute Vermessung vorgenommen wird. Dieses Verfahren kann allerdings einen erheblichen Zeitaufwand erfordern.

FR 2 802 795 A1 offenbart eine Vorrichtung zur Kontrolle der Körperhaltung, bei der mehrere, beispielsweise vier Auflageflächen vorgesehen sind. Die Auflageflächen können einzeln höhenverstellbar sein. Hierzu wirkt eine motorisch angetriebene Spindel auf eine Tragplatte zur Höhenverstellung ein. Zwischen der Tragplatte und der Auflagefläche ist ein Kraftaufnehmer angeordnet. Mit einer derartigen Vorrichtung lässt sich somit die Gewichtsverteilung auf die Auflageflächen bei unterschiedlichen Höheneinstellungen feststellen. Erforderliche Korrekturmaßnahmen können allein aus diesen Werten nicht abgeleitet werden, da die Gewichtsverteilung mit der beobachteten Körperhaltung in Beziehung gebracht werden muss.

Der vorliegenden Erfindung liegt daher die Aufgabe zugrunde, eine Messvorrichtung der eingangs erwähnten Art so auszubilden, dass eine verbesserte und vereinfachte Durchführung von Korrektur- oder Optimierungsmaßnahme möglich ist.

Zur Lösung dieser Aufgabe ist erfindungsgemäß eine Messvorrichtung der eingangs erwähnten Art dadurch gekennzeichnet, dass unterhalb der Auflageflächen und oberhalb der Tragplatte Hubeinrichtungen angeordnet sind.

Die erfindungsgemäße Messvorrichtung bietet somit den Vorteil, dass mittels der Hubeinrichtungen Stellungskorrekturen auf der Messvorrichtung selbst vorgenommen werden können und für die Stellungskorrekturen sofort eine Kontrolle in Form einer Veränderung des durch Messung erzeugen Anzeigewerts der Anzeigeeinrichtung erfolgt. Eine Stellungskorrektur der auf den Auflageflächen stehenden Person wird somit sofort anhand der Veränderung der angezeigten Schwerpunktlinie in ihrer Wirkung erkennbar. Auf diese Weise wird die Ermittlung optimaler Stellungskorrekturen erheblich vereinfacht und beschleunigt. Die Ermittlung geeigneter Stellungskorrekturen ist somit objektivierbar und nicht mehr von der Erfahrung beispielsweise eines Orthopädiemechanikers allein abhängig, der bisher den Grad der Korrektur bzw. Optimierung aufgrund seiner Erfahrung bestimmen musste.

Die Hubeinrichtungen sind vorzugsweise stufenlos verstellbar und vorzugsweise einzeln verstellbar ausgebildet, insbesondere mit einem elektrischen Verstellantrieb.

Die Auflageflächen sind zweckmäßigerweise so ausgerichtet, dass sie die Füße der Person unmittelbar stützen, also beidseitig einer Mittenebene positioniert sind. Die Mittenebene kann dabei sowohl der Frontalebene als auch der Sagittalebene der auf den Auflageflächen stehenden Person entsprechen.

Die Hubeinrichtungen sind vorzugsweise so ausgebildet, dass mit ihnen nicht nur eine auf einen Fuß der stehenden Person wirkende Höhenverstellung möglich ist, sondern auch ein Auflagewinkel parallel oder quer zur Mittenebene variierbar ist, sodass auch die Wirkung von Winkelvariationen des Stehwinkels in Frontalebene oder Sagittalebene kontrollierbar sind.

Es ist zweckmäßig, wenn die Hubeinrichtungen für die Auflageflächen symmetrisch zur Mittenebene angeordnet sind. Dabei können jeweils zwei Hubeinrichtungen auf beiden Seiten der Mittenebene vorhanden sein. Eine größere Variation der Winkelverstellungen ist dadurch möglich, dass auf beiden Seiten der Mittenebene jeweils mindestens drei Hubeinrichtungen vorhanden sind.

Unter Berücksichtigung der Tatsache, dass die Messvorrichtung insbesondere für Gehbehinderte, beispielsweise amputierte Personen geeignet sein soll, ist es wesentlich, die Hubeinrichtungen der Messvorrichtung so flach wie möglich zu bauen, damit das Betreten der Messvorrichtung auch durch gehbehinderte Personen möglich ist und nicht größere Turnübungen erfordert.

Ein besonders flacher Aufbau der Hubeinrichtungen ist dadurch möglich, dass die Hubeinrichtungen aus einer zentralen hohlen Spindelschraube bestehen, die im Eingriff mit wenigstens einem drehfest angeordneten und ein mit der Spindelschraube zusammenwirkendes Innengewinde aufweisenden Hülsenteil steht. Das Hülsenteil kann eine Stirnwand aufweisen, die unmittelbar ein Teil der Auflagefläche bildet. Der durch die Hubeinrichtung bewirkte Hub kann noch dadurch vergrößert werden, dass sie ein oberes und ein unteres drehfest gelagertes Hülsenteil aufweist, die jeweils mit gegenläufigen Außengewindeabschnitten der Spindelschraube zusammenwirken.

Die Spindelschrauben weisen einen Durchmesser auf, der größer als 5 cm, vorzugsweise größer als 10 cm ist. Bevorzugt ist dabei, wenn ein Antrieb der Spindelschraube mit einer im Innern der Spindelschraube angeordneten und mit einer Innenverzahnung der Spindelschraube im Eingriff stehenden Zahnkranzanordnung gebildet ist. Vorzugsweise ist die Zahnkranzanordnung nach Art eines Planetengetriebes ausgebildet. Die kompakte Ausgestaltung der Hubeinrichtungen ermöglicht es auch, einen Antriebsmotor der Zahnkranzanordnung im Innern der Spindelschraube anzuordnen, sodass eine äußerst kompakte Messvorrichtung zur Verfügung steht.

Die Anzeige der Schwerpunktsebene kann bei der erfindungsgemäßen Messvorrichtung mittels eines linear oszillierenden Laserstrahls oder eines durch eine geeignete Linsenoptik, insbesondere eine Zylinderlinse, linienförmig ausgebildeten Lichtstrahls erfolgen. Der Öffnungswinkel des Lichtstrahls kann daher vorzugsweise > 90° betragen.

Die Erfindung soll im Folgenden anhand von in der Zeichnung dargestellten Ausführungsbeispielen näher erläutert werden. Es zeigen:
- Figur 1: eine schematische Darstellung eines Ausführungsbeispiels einer erfindungsgemäßen Messvorrichtung in einer Anwendung zur Bestimmung der frontalen Körperstatik
- Figur 2: die Anordnung gemäß Figur 1 in der Anwendung zur Bestimmung der Statik der unteren Extremität in der Sagittalebene
- Figur 3: eine Draufsicht auf vier säulenförmige Hubeinrichtungen, die Auflageflächen bilden
- Figur 4: eine Seitenansicht der Anordnung gemäß Figur 3
- Figur 5: eine schematische Draufsicht auf zwei durch Platten gebildete Auflageflächen mit jeweils drei darunter befindlichen Hubeinrichtungen
- Figur 6: eine Seitenansicht der Anordnung gemäß Figur 5
- Figur 7: eine schematische Seitenansicht des Aufbaus an einer bevorzugten, kompakt ausgebildeten Hubeinrichtung mit einer hohlen Spindelschraube
- Figur 8: eine Draufsicht auf die hohle Spindelschraube gemäß Figur 7.

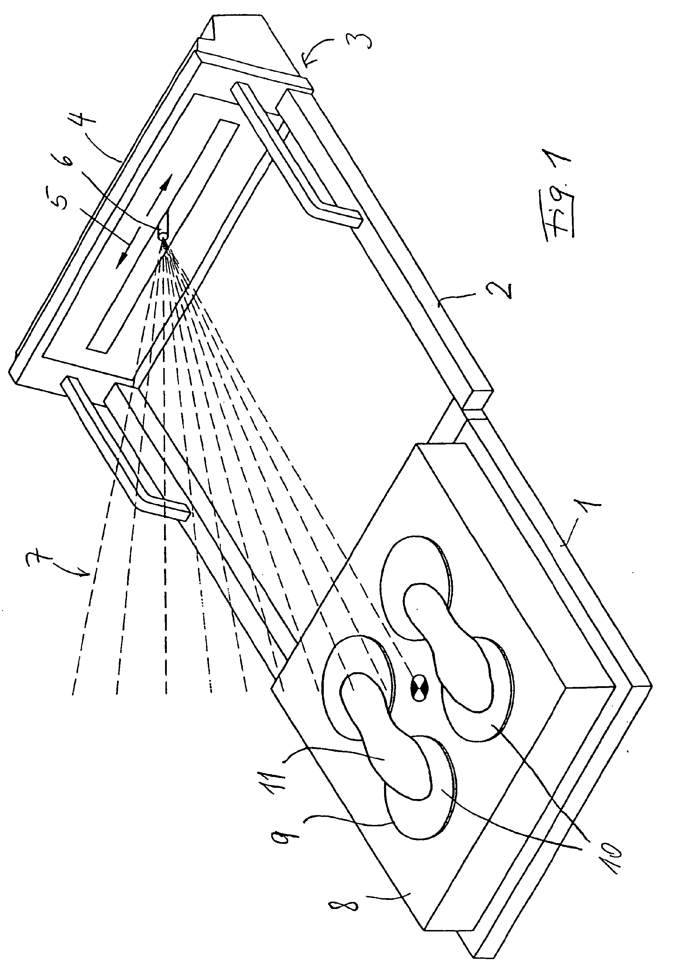

Figur 1 lässt eine Messvorrichtung erkennen, die eine rechteckige Tragplatte aufweist, die auf Druck-, Kraft- oder Wegsensoren, vorzugsweise in den Ecken der Tragplatte 1 angeordnet, gelagert ist. Über nicht dargestellte elektrische Verbindungen, die durch Rahmenteile 2 eines Gehäuses 3 verlaufen, sind die Sensoren der Tragplatte 1 mit einer Elektronik in einem stirnseitigen Abschlussteil 4 des Gehäuses 3 verbunden. In dem stirnseitigen Abschlussteil 4 des Gehäuses 3 ist ein in Richtung der eingezeichneten Pfeile 5 seitlich verfahrbarer Schlitten gelagert, an dem eine Projektionsoptik 6 angebracht ist, mit der ein Strahl eines (nicht dargestellten) Lasers senkrecht zum stirnseitigen Abschlussteil 4 des Gehäuses 3 projiziert werden kann. Wie in Figur 1 angedeutet ist, oszilliert die Ablenkoptik 6 in vertikaler Richtung, also senkrecht zur Oberfläche der Tragplatte 1 in einem Winkelbereich von beispielsweise 30° und spannt somit mit einem oszillierenden Laserstrahl 7 einen Teil einer gemessenen Schwerpunktsebene auf. Je nach der gemessenen Schwerkrafteinwirkung verfährt der Schlitten 6 soweit seitlich, dass der oszillierende Laserstrahl 7 sich im Bereich der Schwerpunktsebene einer auf der Tragplatte 1 stehenden Person befindet.

Oberhalb der Tragplatte 1 ist erfindungsgemäß ein Aufbau 8 vorgesehen, der gemäß Figur 1 vier Hubeinrichtungen 9 aufweist. Die Hubeinrichtungen 9 sind in einer quadratischen Anordnung so positioniert, dass sie gemeinsam eine Auflagefläche 10 bilden, auf der schematisch angedeutete Füße 11 einer Person stehen können.

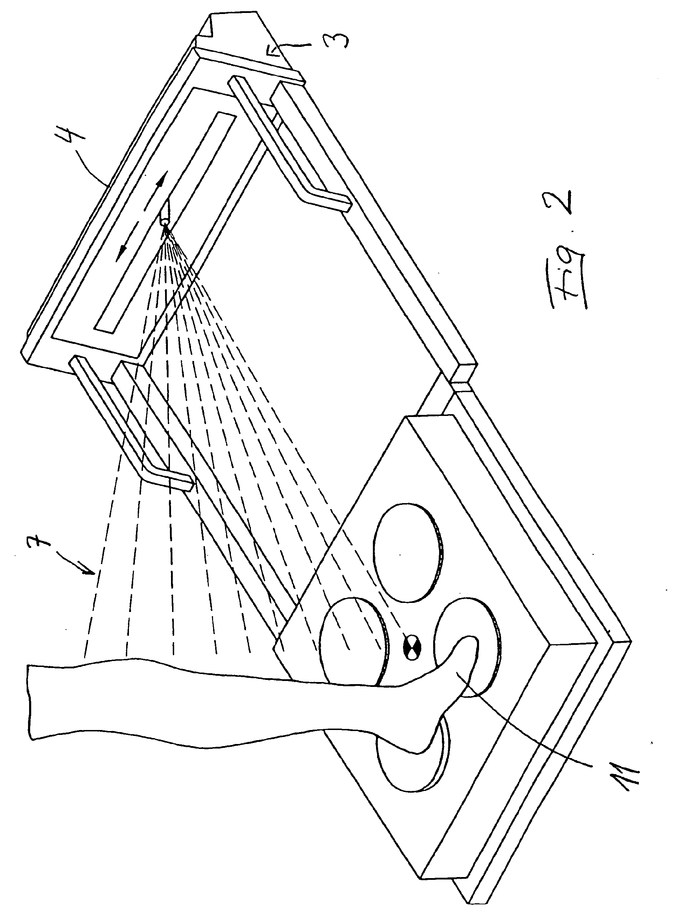

Die Position der Füße 11 einer Person ist in Figur 1 so dargestellt, dass der oszillierende Laserstrahl 7 eine Schwerpunktsebene für die frontale Körperstatik projiziert. Demgegenüber ist für die in Figur 2 dargestellte gleiche Anordnung eine Belastung mit einem Fuß 11 einer Person angedeutet, die seitlich zum stirnseitigen Abschlussteil 4 des Gehäuses 3 steht, sodass der oszillierende Laserstrahl 7 eine Schwerpunktsebene in der Sagittalebene der Person aufspannt.

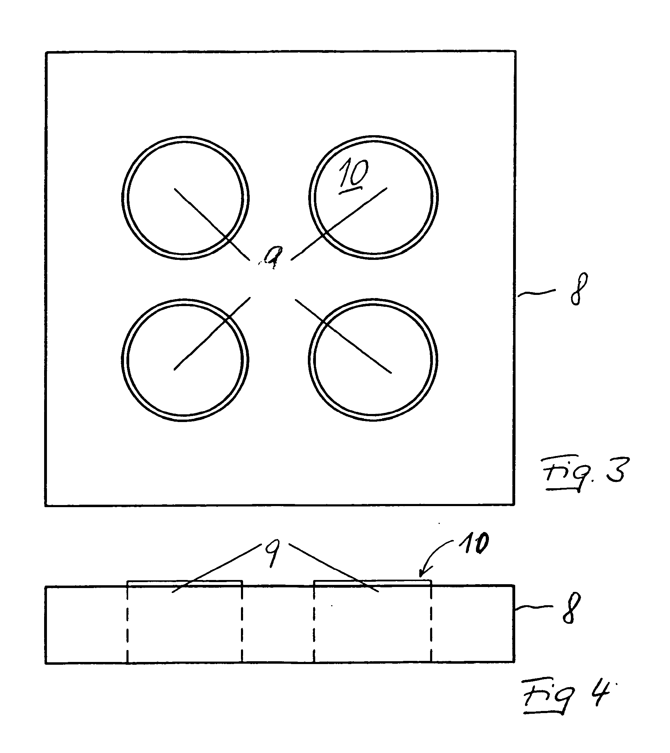

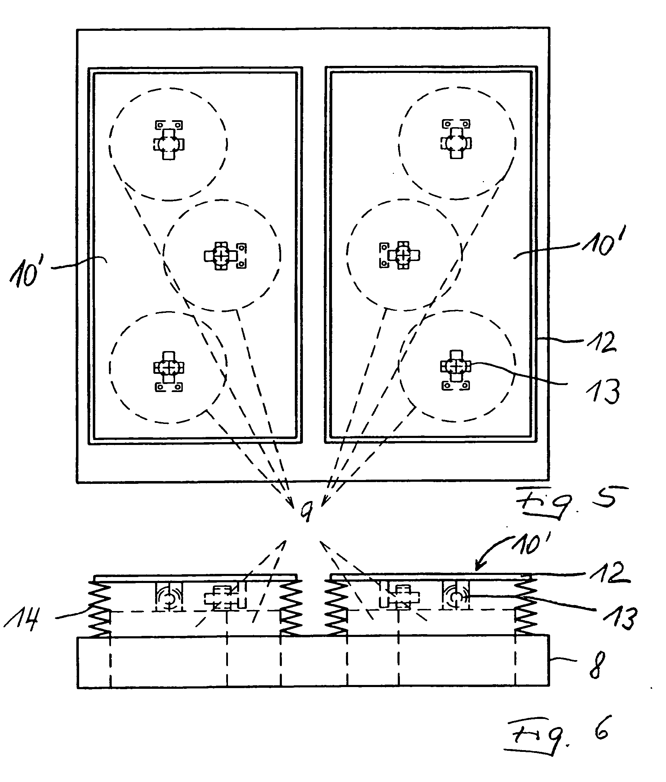

Die Figuren 3 und 4 verdeutlichen in einer separaten Darstellung des Aufbaus 8 die Hubeinrichtungen 9, die die Auflageflächen 10 für die Füße 11 einer Person bilden.

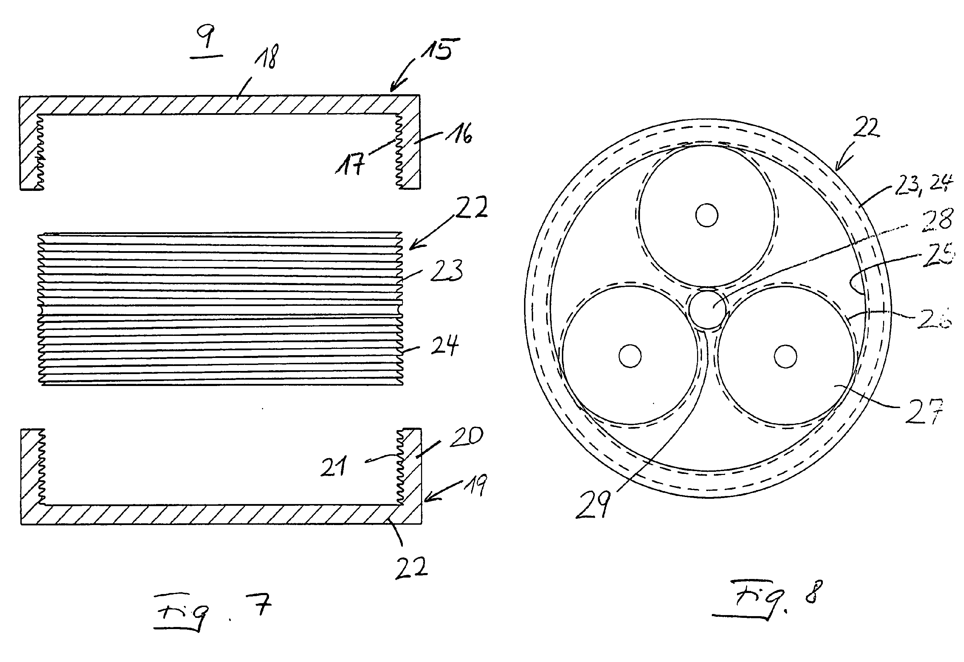

Demgegenüber zeigen die Figuren 5 und 6 eine Ausführungsform, bei der Auflageflächen 10' durch jeweils eine rechteckige Platte 12 gebildet sind, die oberhalb von hier jeweils drei Hubeinrichtungen 9 angeordnet ist. Die Platte 12 ist jeweils mit Drehgelenken 13 mit den Hubeinrichtungen 9 verbunden, um eine Kippung der Platten 12 zu ermöglichen, die zu einer Variation des Winkels der Auflageflächen 10' führt. Es ist erkennbar, dass die Platten 12 so gekippt werden können, dass sich ein Winkel in Längsrichtung oder ein Kippwinkel der Auflagefläche 10' in Querrichtung oder eine Kombination davon ausbildet. Der Zwischenraum zwischen der Platte 12 und dem Aufbau 8 kann zweckmäßigerweise mit einem Faltenbalg 14 abgedichtet werden.

Die Figuren 7 und 8 verdeutlichen einen bevorzugten Aufbau der Hubeinrichtungen 9. Diese bestehen gemäß Figur 7 aus einem oberen drehfest angeordneten Hülsenteil 15, dass eine zylindrische Mantelwand 16 mit einem Innengewinde 17 und eine obere Stirnwand 18 aufweist. In entsprechender Weise ist eine unteres Hülsenteil 19 mit einer zylindrischen Mantelwand 20 mit einem Innengewinde 21 und mit einer unteren Stirnwand 22 ausgebildet. Beide Hülsenteile 15, 19 wirken mit einer zentralen hohlen Spindelschraube 22 zusammen, die über ihre Höhe mit zwei gleich großen, gegenläufigen Außengewindeabschnitten 23, 24 versehen ist. Befinden sich die beiden Hülsenteile 15, 19 mit der Spindelschraube 22 im Wirkeingriff, führt eine Drehung der Spindelschraube 22 in die eine Richtung dazu, dass sich die beiden Hülstenteile 15, 19 von einer radialen Mittenebene der Spindelschraube weg bewegen, während die Drehung der Spindelschraube 22 in der entgegengesetzten Richtung zu einer Bewegung der Hülsenteile 15, 19 in Richtung der radialen Mittenebene führt. Auf diese Weise bewegen sich die beiden Stirnseiten 18, 22 aufeinander zu oder voneinander weg. Steht die Hubeinrichtung auf der unteren Stirnwand 22, bewegt sich somit die obere Stirnwand 18 in Abhängigkeit von der Drehung der Spindelschraube 22 nach oben oder nach unten.

Der Antrieb der Spindelschraube 22 selbst ist in Figur 8 schematisch dargestellt. Die Spindelschraube 22 ist hohl ausgeführt und weist auf ihrer zylindrischen Innenwandung eine Innenverzahnung 25 auf, in die Außenverzahnungen 26 dreier sternförmig angeordneter Zahnräder 27 eingreifen. Die drei Zahnräder 27 werden durch eine zentrale Antriebswelle 28 mit einer Außenverzahnung 29 angetrieben.

Die zentrale Antriebswelle 28 kann direkt mit einem ebenfalls im Innern der Spindelschraube 22 angeordneten Elektromotor verbunden sein bzw. die Abtriebswelle des Elektromotors bilden.

Der in den Figuren 7 und 8 dargestellte Aufbau einer Hubeinrichtung ermöglicht einen Aufbau 8 mit einer geringen Bauhöhe, wobei die Oberseite der oberen Stirnwand 18 gemäß Figur 1 unmittelbar die Auflagefläche 10 bilden kann.

## Patentansprüche

1. Messvorrichtung mit einer auf Messzellen gelagerten Tragplatte (1) und einer Anzeigeeinrichtung zur Anzeige einer Kraftwirkungslinie (7) für eine auf Auflageflächen (10, 10') oberhalb der Tragplatte (1) stehenden Person, **dadurch gekennzeichnet, dass** unterhalb der Auflageflächen (10, 10') und oberhalb der Tragplatte (1) Hubeinrichtungen (9) angeordnet sind.

2. Messvorrichtung nach Anspruch 1, **dadurch gekennzeichnet, dass** die Hubeinrichtungen (9) stufenlos verstellbar ausgebildet sind.

3. Messvorrichtung nach Anspruch 1, **dadurch gekennzeichnet, dass** die Hubeinrichtungen (9) einzeln verstellbar ausgebildet sind.

4. Messvorrichtung nach einem der Ansprüche 1 bis 3, **dadurch gekennzeichnet, dass** die Hubeinrichtungen (9) elektrisch verstellbar ausgebildet sind.

5. Messvorrichtung nach einem der Ansprüche 1 bis 4, **dadurch gekennzeichnet, dass** die Auflageflächen (10, 10') parallel zueinander beidseitig einer Mittenebene angeordnet sind.

6. Messvorrichtung nach Anspruch 5, **dadurch gekennzeichnet, dass** die Auflageflächen (10, 10') mittels der Hubeinrichtungen (9) in einem Auflagewinkel parallel zur Mittenebene variierbar sind.

7. Messvorrichtung nach Anspruch 5 oder 6, **dadurch gekennzeichnet, dass** die Auflageflächen (10, 10') mittels der Hubeinrichtungen (9) in einem Auflagewinkel quer zur Mittenebene varrierbar sind.

8. Messvorrichtung nach einem der Ansprüche 5 bis 7, **dadurch gekennzeichnet, dass** die Hubeinrichtungen (9) für die Auflageflächen (10, 10') symmetrisch zur Mittenebene angeordnet sind.

9. Messvorrichtung nach einem der Ansprüche 5 bis 8, **dadurch gekennzeichnet, dass** jeweils zwei Hubeinrichtungen (9) auf beiden Seiten der Mittenebene vorhanden sind.

10. Messvorrichtung nach einem der Ansprüche 5 bis 9, **dadurch gekennzeichnet, dass** jeweils mindestens drei Hubeinrichtungen (9) auf beiden Seiten der Mittenebene vorhanden sind.

11. Messvorrichtung nach einem der Ansprüche 1 bis 10, **dadurch gekennzeichnet, dass** die Hubeinrichtungen (9) aus einer zentralen hohlen Spindelschraube (22) bestehen, die im Eingriff mit wenigstens einem drehfest angeordneten und ein mit der Spindelschraube (22) zusammenwirkendes Innengewinde (17, 21) aufweisenden Hülsenteil (15, 19) steht.

12. Messvorrichtung nach Anspruch 11, **dadurch gekennzeichnet, dass** ein Antrieb der Spindelschraube (22) mit einer im Innern der Spindelschraube (22) angeordneten und mit einer Innenverzahnung (25) der Spindelschraube (22) im Eingriff stehenden Zahnkranzanordnung (27, 28) gebildet ist.

13. Messvorrichtung nach Anspruch 12, **dadurch gekennzeichnet, dass** die Zahnkranzanordnung (27, 28) eine Planetengetriebeanordnung aufweist.

14. Messvorrichtung nach Anspruch 12 der 13, **dadurch gekennzeichnet, dass** auch ein Antriebsmotor der Zahnkranzanordnung (27, 28) im Innern der Spindelschraube (22) angeordnet ist.

15. Messvorrichtung nach einem der Ansprüche 10 bis 14, **dadurch gekennzeichnet, dass** die Hubeinrichtung (9) ein oberes und ein unteres drehfest gelagertes Hülsenteil (15, 19) aufweist, die mit gegenläufigen Außengewindeabschnitten (23, 24) der Spindelschraube (22) zusammenwirken.

16. Messvorrichtung nach einem der Ansprüche 11 bis 15, **dadurch gekennzeichnet, dass** die Spindelschraube (22) einen Durchmesser von größer 5 cm, vorzugsweise größer 10 cm, aufweist.

17. Messvorrichtung nach einem der Ansprüche 1 bis 16, **dadurch gekennzeichnet, dass** die Anzeige einer Schwerpunktlinie durch Projektion eines Teils einer Schwerpunktebene über der Tragplatte (1) gebildet ist.

18. Messvorrichtung nach Anspruch 17, **dadurch gekennzeichnet, dass** zur Projektion des Teils der Schwerpunktebene ein senkrecht zur Tragplatte (1) oszilierender Laserstrahl (7) vorgesehen ist.

19. Messvorrichtung nach Anspruch 17, **dadurch gekennzeichnet, dass** zur Projektion des Teils der Schwerpunktebene ein Lichtstrahl mittels einer Linsenoptik linienförmig ausgebildet ist.

## Claims

1. A measurement device with a support plate (1) mounted on measurement cells, and with a display device for displaying a force action line (7) for a person standing on bearing surfaces (10, 10') above the support plate (1), wherein lifting devices (9) are arranged underneath the bearing surfaces (10, 10') and above the support plate (1).

2. The measurement device as claimed in claim 1, wherein the lifting devices (9) are designed to be steplessly adjustable.

3. The measurement device as claimed in claim 1, wherein the lifting devices (9) are designed to be individually adjustable.

4. The measurement device as claimed in one of claims 1 through 3, wherein the lifting devices (9) are designed to be electrically adjustable.

5. The measurement device as claimed in one of claims 1 through 4, wherein the bearing surfaces (10, 10') are arranged parallel to one another on both sides of a center plane.

6. The measurement device as claimed in claim 5, wherein the bearing surfaces (10, 10') can be varied by means of the lifting devices (9) in a bearing angle parallel to the center plane.

7. The measurement device as claimed in claim 5 or 6, wherein the bearing surfaces (10, 10') can be varied by means of the lifting devices (9) in a bearing angle transverse to the center plane.

8. The measurement device as claimed in one of claims 5 through 7, wherein the lifting devices (9) for the bearing surfaces (10, 10') are arranged symmetrically with respect to the center plane.

9. The measurement device as claimed in one of claims 5 through 8, wherein two lifting devices (9) are in each case present on both sides of the center plane.

10. The measurement device as claimed in one of claims 5 through 9, wherein at least three lifting devices (9) are in each case present on both sides of the center plane.

11. The measurement device as claimed in one of claims 1 through 10, wherein the lifting devices (9) consist of a central hollow spindle screw (22) in engagement with at least one sleeve part (15, 19) which is rotationally fixed and has an internal thread (17, 21) cooperating with the spindle screw (22).

12. The measurement device as claimed in claim 11, wherein a drive mechanism of the spindle screw (22) is formed with a toothed ring arrangement (27, 28) arranged in the inside of the spindle screw (22) and in engagement with an internal toothing (25) of the spindle screw (22).

13. The measurement device as claimed in claim 12, wherein the toothed ring arrangement (27, 28) has a planetary gear configuration.

14. The measurement device as claimed in claim 12 or 13, wherein a drive motor of the toothed ring arrangement (27, 28) is also arranged in the inside of the spindle screw (22).

15. The measurement device as claimed in one of claims 10 through 14, wherein the lifting device (9) has upper and lower rotationally fixed sleeve parts (15, 19) which cooperate with oppositely directed external thread sections (23, 24), respectively, of the spindle screw (22).

16. The measurement device as claimed in one of claims 11 through 15, wherein the spindle screw (22) has a diameter of greater than 5 cm, preferably greater than 10 cm.

17. The measurement device as claimed in one of claims 1 through 16, wherein the display of a line of the center of gravity is formed by projection of part of a plane of the center of gravity over the support plate (1).

18. The measurement device as claimed in claim 17, wherein a laser beam (7) oscillating perpendicularly with respect to the support plate (1) is provided in order to project part of the plane of the center of gravity.

19. The measurement device as claimed in claim 17, wherein, in order to project part of the plane of the center of gravity, a light beam is formed linearly by means of a lens optical system.

## Revendications

1. Appareil de mesure comportant une plaque de support (1) disposée sur des cellules de mesure et un dispositif d'affichage pour afficher une ligne d'effet de force (7) pour une personne se tenant debout sur des surfaces de réception (10, 10') au dessus de la plaque de support (1) **caractérisé en ce que** des dispositifs de levage (9) sont disposés en dessous des surfaces de réception (10, 10') et au dessus de la plaque de support (1).

2. Appareil de mesure selon la revendication 1, **caractérisé en ce que** les dispositifs de levage (9) sont réalisés pour être mobiles sans étapes.

3. Appareil de mesure selon la revendication 1, **caractérisé en ce que** les dispositifs de levage (9) sont réalisés pour être mobiles individuellement.

4. Appareil de mesure selon l'une des revendications 1 à 3, **caractérisé en ce que** les dispositifs de levage (9) sont réalisés pour être mobiles électriquement.

5. Appareil de mesure selon l'une des revendications 1 à 4, **caractérisé en ce que** les surfaces de réception (10, 10') sont disposées parallèlement les unes par rapport aux autres de part et d'autre d'un plan médian.

6. Appareil de mesure selon la revendication 5, **caractérisé en ce que** les surfaces de réception (10, 10') sont réglables au moyen des dispositifs de levage (9) selon un angle de réception parallèle au plan médian.

7. Appareil de mesure selon la revendication 5 ou 6, **caractérisé en ce que** les surfaces de réception (10, 10') sont réglables au moyen des dispositifs de levage (9) selon un angle de réception transversal au plan médian.

8. Appareil de mesure selon l'une des revendications 5 à 7, **caractérisé en ce que** les dispositifs de levage (9) pour les surfaces de réception (10, 10') sont disposés symétriquement par rapport au plan médian.

9. Appareil de mesure selon l'une des revendications 5 à 7, **caractérisé en ce que** deux dispositifs de levage (9) sont présents sur chacun des deux côtés du plan médian.

10. Appareil de mesure selon l'une des revendications 5 à 8, **caractérisé en ce qu'**au moins trois dispositifs de levage (9) sont présents sur chacun des deux côtés du plan médian.

11. Appareil de mesure selon l'une des revendications 1 à 10, **caractérisé en ce que** les dispositifs de levage (9) sont constitués par une broche filetée creuse (22) qui est en prise de façon fixe en rotation avec le filetage intérieur (17, 21) d'au moins une partie de manchon (15, 19) coopérant avec la broche filetée (22).

12. Appareil de mesure selon la revendication 11, **caractérisé en ce qu'**un entraînement de la broche filetée (22) est réalisé par un agencement d'engrenages (27, 28) disposés en croix à l'intérieur de la broche filetée (22) en prise avec un filetage intérieur (25) de la broche filetée (22).

13. Appareil de mesure selon la revendication 12, **caractérisé en ce que** l'agencement d'engrenages (27, 28) présente un agencement d'engrenages planétaires.

14. Appareil de mesure selon l'une des revendications 12 ou 13, **caractérisé en ce qu'**en outre un moteur d'entraînement de l'agencement d'engrenages (27, 28) est disposé à l'intérieur de la broche filetée (22).

15. Appareil de mesure selon l'une des revendications 10 à 14, **caractérisé en ce que** le dispositif de levage (9) présente une partie supérieure et inférieure de manchon (15, 19) montées de façon fixe en rotation qui coopèrent avec des sections de filetages extérieurs (23, 24) de la broche filetée (22) ayant des sens contraires.

16. Appareil de mesure selon l'une des revendications 11 à 15, **caractérisé en ce que** la broche filetée (22) présente un diamètre extérieur supérieur à 5cm de préférence supérieur à 10cm.

17. Appareil de mesure selon l'une des revendications 1 à 16, **caractérisé en ce que** l'affichage d'une ligne de points de poids est réalisé par projection d'une partie d'un plan de points de poids sur la plaque de support (1).

18. Appareil de mesure selon la revendication 17, **caractérisé en ce que** pour la projection de la partie du plan de points de poids est prévu un faisceau laser (7) oscillant perpendiculairement à la plaque de support (1).

19. Appareil de mesure selon la revendication 17, **caractérisé en ce que** pour la projection de la partie du plan de points de poids est prévu un faisceau de lumière formé de façon linéaire avec une optique à lentilles.
